# EUROPEAN PATENT APPLICATION

(11) **EP 2 050 426 A1**
(43) Date of publication of application: **22.04.2009**
(21) Application number: 07301481.3
(22) Date of filing: 19.10.2007
(51) Int. Cl.: A61G 12/00, G08B 21/04

(54) **Active floor mate to monitor sick person**

(71) Applicant: INSERM (Institut National de la Santé et de la Recherche Médicale), 75654 Paris Cedex 13 (FR)
(72) Inventor: Steenkeste, François, 11270, Laurac Le Grand (FR)
(74) Representative: Cabinet Plasseraud

(57) **Abstract**

A Floor mat comprises at least one presence sensor (5, 7, 9, 13) of a human body, the sensor being adapted to detect a pressure variation on the floor mat and a body presence near the sensor.

## Description

### Field of the invention

The invention relates to the field of active floor mate.

### Background of the invention

When a sick person is confided to bed due to his/her illness, there is a need for nurses and hospital personnel to be notified when the person falls from the bed or goes away from it. This need is particularly significant when the person is excited or gets mad as with Alzheimer illness, for instance.

To answer this need, different systems have been developed. For instance, the patent application JP2005185812 discloses a mat placed on the floor around the bed. The mat contains a pressure sensitive sensor such that, when a person falls down from the bed, the pressure sensitive sensor informs a contact site and a monitor displays an image of the bed taken by a monitoring camera.

The patent application JP6168391 discloses a floor mat installed on the floor of a lavatory room and divided into plural floor mat components. The lavatory room contains also a thermal ray type detector. The system judges that fall-down is generated in the lavatory room when more than two of the plural mat components send continuous human body pressure detection signals for more than a prescribed time and after recognizing that the thermal ray type detector transmits human body heat detection signals. Thus, there is less probability that more than a prescribed number of the plural mat components transmit the human body pressure detection signals even when hand luggage or the like is left alone on the floor of the room. Thus, the system hardly issues a false fall-down alarm.

However, these systems do not detect the leave of a person as the pressure variation on the mat generated by the feet of a person who sits on the bed, then stands up and then walks is much lower than the pressure variation generated by a fall. Further more, these systems need a mix of sensors, floor mat and camera or thermal ray detector, which are costly and often difficult to setup.

### Summary of the invention

It would advantageous to achieve a system able to detect the fall, and/or the leave, of a sick person from his/her bed, which is inexpensive and easy to setup. To better address one or more concerns, in a first aspect of the invention a floor mat comprises at least one presence sensor of a human body, characterized in that the sensor is adapted to detect a pressure variation on the floor mat and a body presence near the sensor.

By combining two types of detection, i.e. pressure variation and presence detection, the floor mate has the advantage to be very sensitive whilst reducing false alarm. In particular embodiment:
- each presence sensor comprises a capacitor comprising two conductive and flexible layers and a dielectric and resilient intermediate layer between the two conductive layers, said intermediate layer being deformed by a pressure exercised on the tile.
- each conductive layer for all the sensors is above 50% of the floor mat area.
- the sensor further comprises computing means adapted to detect a variation of the capacitor capacitance related to the deformation of the intermediate layer.
- the sensor further comprises computing means adapted to detect a charge transfer in the capacitor related to the body presence.
- the sensor further comprises communication means connected to a centralized control unit to send information of pressure variation and/or body presence with an identification code of the sensor.
- the sensor further comprises means to disconnect and/or calibrate it on request of the centralized control unit.
- the centralized control unit for receiving information of pressure variation and/or body presence from said tiles and for generating a control signal to alarm and/or supervision means.
- the centralized control unit is adapted to send an alarm signal when it received information of pressure variation and/or body presence from a number of contiguous tiles above a predetermined number.
- the centralized control unit is adapted to send to the supervision means information on the course of a person by analysis of the location of the tiles which send information of pressure variation and/or body presence as a time function.
- the centralized control unit is adapted to send to any tile of the floor mat a request to deactivate or to calibrate the sensor.
- it comprises a plurality of sensors organized in a regular network.

Advantageously, the mat is easy to manufacture as it does not require complex materials. By using a single capacitor in two detection modes, it reduces the cost of the mat whilst being sensitive to both a pressure and a presence.

Advantageously, the identification of each sensor increases the quality of the detector as it defines the area of the mat where the body is detected. For instance, a pressure variation just around a bed has a greater probability to indicate the fall of a sick person than a detection near the door of the room which may indicate the arrival of a nurse.

Advantageously, the centralized control unit may modify, or suppress the sensor detection, in part or in whole, which adapt the mat to its environment. For instance, the mat may be disabled during nursing or visit. Or some part of the mat may be calibrated to react to higher, or lower, pressure variation. The alarm detection may also be adapted by defining the number of sensors which must record a presence or a pressure variation.

These and other aspects of the invention will be apparent from and elucidated with reference to the embodiment described hereafter where:
- Figure 1 is a schematic and isometric view of a tile according to an embodiment of the invention; and
- Figure 2 is a schematic view of a mat composed of tiles of Figure 1.

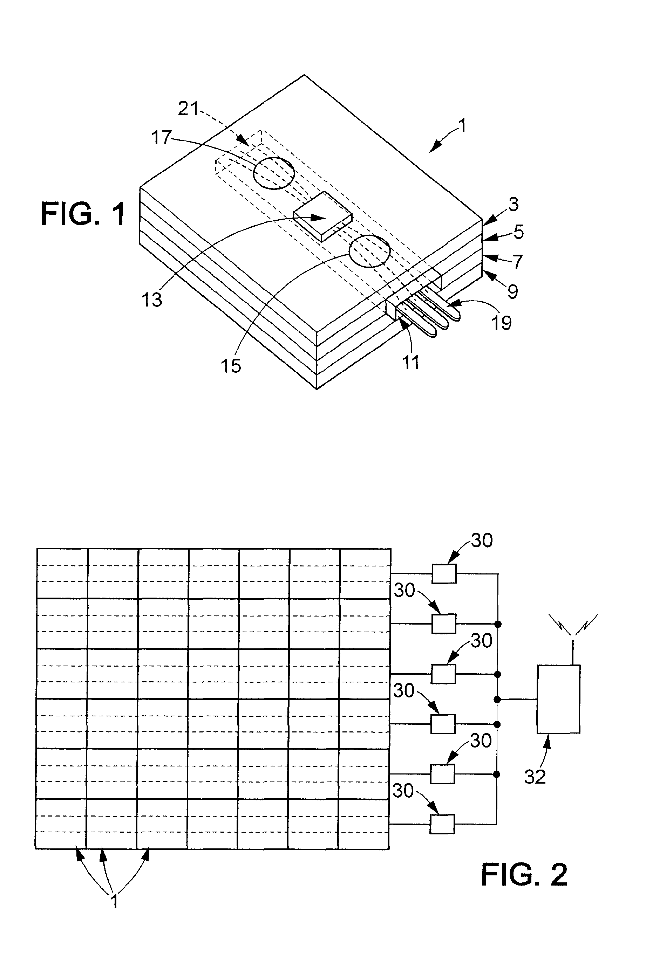

In reference to Figure 1, a floor tile 1 has a square shape of a standardized size of 50 x 50, or 30 x 30, square centimeter. It comprises four layers which are stacked up. The upper layer 3 is a wear and tear layer to protect the tile from person walk, dirt, etc and is made of plastic, for instance PVC, or any other suitable material. It protects an electrically conductive layer 5 which, with a dielectric layer 7 and a second electrically conductive layer 9 forms a capacitor. Optionally, a lower dielectric layer (not represented) may be used to protect the floor tile from the floor on which the tile 1 is put.

The dielectric layer 7 is composed of a flexible material such as foam.

So the dielectric layer 7 and the two conductive layers 5, 9 create a capacitor of which the capacitance is modified when a pressure is exercised on the tile 1.

As an example, with foam having a thickness of 3 millimeters and a density of 0.15, the capacitance varies from 5 to 16 % of a reference value for a weight pressing the tile of 4 to 75 kg.

The dielectric layer 7 comprises a void 11 in which a microcontroller 13 is fixed up. Two electrical cables 15, 17, such as ribbon cables, connect electrically the microcontroller 13 to two connectors 19, 21 disposed on two opposite sides of the tile 1. Preferably, the two connectors are of the male or female type each so that two contiguous tiles may be electrically connected together.

The microcontroller 13 is connected to the capacitor of the tile to form a presence sensor of the tile.

To detect the presence of a human body, the sensor comprises two working modes: a pressure variation mode and a proximity mode.

The pressure variation exercised by a part of a human body on the tile modifies the thickness of the dielectric layer 7 what generates a capacitance variation which is detected by the microcontroller 13.

The proximity mode is based on the charge transfer generates by the human body. When a part of a human body approaches to the tile, it generates in the upper conductive layer 5 a movement of the electric charges which is detected by the microcontroller 13.

These two working modes are complementary: to avoid false detection due to small electrical variations, the pressure variation detection is limited to variation above a predetermined value. However, this limitation inhibits the detection of small weight on the tile. For instance, an elderly person who falls from his/her bed to several tiles may generate only a small variation pressure on each tile as his/her weight is spread on different tiles.

At the opposite, a human body near the tile, at few millimeters, generates a charge transfer whatever the human body weight.

To form an active floor mat, a regular network of the floor tiles is formed, Figure 2.

On each row, the tiles are connected together electrically through the connectors up to a row controller 30. And the row controllers 30 are connected to a central controller 32.

The electrical connections between tiles create a bus to distribute the electrical power to the tiles and to transmit information from/to each tile to the row controller 30. Particularly, each tile is able to transmit presence information with an identification code of the tile so that the row controller and/or the central controller can know which tile is sending presence information.

The row controller and/or the central controller use the bus to transmit to a tile requests to calibrate, to connect or to disconnect.

The central controller 32 centralizes all information coming from the tiles and generates control signal to alarm and/or supervision means.

By analyzing information coming from tiles, the central controller 32 is able to differentiate information generated by a standing person who generates a detection signal in 6 or 8 tiles at most and information generated by a falling person who generates a detection signal in around 20 tiles. The tile size, which is typically 30*30 square centimeters, may be modified to be adapted to the person to monitor. For instance, it may be advantageous to use smaller tiles to monitor children.

The mat may be used also to monitor and study the walking activity of a person. In this case, the central controller contains a map of the tiles to be able to correlate the presence information sent by tiles to their position in the mat. By adding the time information about the detection, the central controller is able to trace the itinerary of a person walking on the mat.

While the invention has been illustrated and described in details in the drawings and foregoing description, such illustration and description are to be considered illustrative or exemplary and not restrictive; the invention is not limited to the disclosed embodiment.

For instance, the tile size and form may vary depending on the type of use. As explained above, if it is necessary to monitor children of to have a precise map of the walking of a person, the mat will be composed of small, for instance 20*20 square centimeters, tiles. At the opposite, if the mat is only used to monitor the fall of an adult, large tiles, for instance 50*50 square centimeters tiles, may be used.

Although the square form has the advantage to define easily the mapping of every tile as a 2D matrix, other regular form may be used such as, for instance, the hexagonal form.

The size of the conductive layers compared to the total size of the tile may vary. However, the ratio conductive layer size / tile size is preferably above 50% to detect a pressure variation or a charge transfer on any part of the tile. To avoid that a contiguous tile generates a charge transfer, the conductive layer has advantageously a size which is less than the tile size so that the distance between the conductive layers of two contiguous tiles avoids an electrical coupling of the conductive layers.

To ease the management of the mat, as explained above, the central controller may send request to a specific tile to calibrate and/or (dis)connect.

The calibration may adjust different parameters of the sensor. For instance, it may modify the predetermined level of detection for pressure variation, the slow drift of the sensor and/or the number of consistent answers before the detection acknowledgement.

As an example of embodiment of these control requests, each request is composed of 5 bytes. The first byte is the control to be executed, the second byte is the row in the mat matrix, the third byte is the tile number in the row, the fourth byte is the parameter value to be modified and the fifth byte is a CRC to validate the transmission of the request. As an acknowledgement, the tile microcontroller sends a five byte answer in which the first byte is the ASCII value of 'V' as Validate, the second, third and fourth bytes are not modified and the fifth byte is a new CRC taking into account the modification of the first byte.

In the described embodiment, each tile has a microcontroller. This embodiment has the advantage to reduce the data flow on the mat bus. However, to reduce cost, it is possible to have only a row controller to control the tiles and to reduce the electronics of each tile to the sensing of pressure variation and charge transfer.

It is also possible to integrate the tiles into the mat, i.e to make a one-piece mat containing a 2D matrix of sensors. Although, this embodiment has the disadvantage to define the mat dimension at the manufacturing step, it has the advantage to reduce the cost of the mat by optimizing the sensor connection (there is no need for connectors for each sensor) and defining an electronic structure optimized for the mat, for instance, by sharing a microcontroller with a plurality of capacitors.

Another embodiment consists of tiles containing a plurality of sensors. For instance, a rectangular tile with two square sensors or a square tile with four square sensors may be manufactured. This embodiment combines some advantages of the single-piece mat in term of cost and optimization and some advantages of the single sensor tiles in term of versatility.

Other variations to the disclosed embodiments can be understood and effected by those skilled on the art in practicing the claimed invention, from a study of the drawings, the disclosure and the appended claims. In the claims, the word "comprising" does not exclude other elements and the indefinite article "a" or "an" does not exclude a plurality.

## Claims

1. Floor mat comprising at least one presence sensor (5, 7, 9, 13) of a human body, **characterized in that** the sensor is adapted to detect a pressure variation on the floor mat and a body presence near the sensor.

2. Floor mat according to claim 1, **characterized in that** each presence sensor comprises a capacitor comprising two conductive and flexible layers (5, 9) and a dielectric and resilient intermediate layer (7) between the two conductive layers, said intermediate layer being deformed by a pressure exercised on the tile.

3. Floor mat according to claim 2, **characterized in that** the total of each conductive layer for all the sensors is above 50% of the floor mat area.

4. Floor mat according to claim 2 or 3, **characterized in that** the sensor further comprises computing means (13) adapted to detect a variation of the capacitor capacitance related to the deformation of the intermediate layer.

5. Floor mat according to one of any claims 2 to 4, **characterized in that** the sensor further comprises computing means (13) adapted to detect a charge transfer in the capacitor related to the body presence.

6. Floor mat according to one of any preceding claims, **characterized in that** the sensor further comprises communication means connected to a centralized control unit (32) to send information of pressure variation and/or body presence with an identification code of the sensor.

7. Floor mat according to claim 6, **characterized in that** the sensor further comprises means to disconnect and/or calibrate it on request of the centralized control unit.

8. Floor mat according to claim 6 or 7, **characterized in that** it further comprises the centralized control unit for receiving information of pressure variation and/or body presence from said tiles and for generating a control signal to alarm and/or supervision means.

9. Floor mat according to claim 8, **characterized in that** the centralized control unit is adapted to send an alarm signal when it received information of pressure variation and/or body presence from a number of contiguous tiles above a predetermined number.

10. Floor mat according to claim 8 or 9, **characterized in that** the centralized control unit is adapted to send to the supervision means information on the course of a person by analysis of the location of the tiles which send information of pressure variation and/or body presence as a time function.

11. Floor mat according to claim 8, 9 or 10, **characterized in that** the centralized control unit is adapted to send to any tile of the floor mat a request to deactivate or to calibrate the sensor.

12. Floor mat according to any preceding claims, **characterized in that** it comprises a plurality of sensors organized in a regular network.
